# EUROPEAN PATENT SPECIFICATION

(11) **EP 3 016 657 B1**
(45) Date of publication and mention of the grant of the patent: **26.06.2019**
(21) Application number: 14819550.6
(22) Date of filing: 02.07.2014
(51) Int. Cl.: A61K 31/496, A61K 9/00, A61P 25/00

(54) **METHOD FOR PREVENTING AND/OR TREATING CHRONIC TRAUMATIC ENCEPHALOPATHY-II**
VERFAHREN ZUR PRÄVENTION UND/ODER BEHANDLUNG VON CHRONISCH TRAUMATISCHER ENZEPHALOPATHIE-II
PROCÉDÉ POUR LA PRÉVENTION ET/OU LE TRAITEMENT DE L'ENCÉPHALOPATHIE TRAUMATIQUE CHRONIQUE-II

(30) Priority: 02.07.2013 AU 2013902457
(43) Date of publication of application: 11.05.2016
(73) Proprietor: Eustralis Pharmaceuticals Limited (Trading as Pressura Neuro), Melbourne, Victoria 3000 (AU)
(72) Inventor: VINK, Robert, Mylor, South Australia 5153 (AU)
(74) Representative: Lavoix
(86) International application number: PCT/AU2014/050108
(87) International publication number: WO 2015/000033

(56) References cited:
- US-A1- 2003 083 345
- US-A1- 2006 247 240
- US-A1- 2009 253 698
- US-A1- 2011 053 954
- JAMES J. DONKIN ET AL: "A Substance P Antagonist Reduces Axonal Injury and Improves Neurologic Outcome When Administered Up to 12 Hours after Traumatic Brain Injury", JOURNAL OF NEUROTRAUMA., vol. 28, no. 2, 1 February 2011 (2011-02-01), pages 217-224, XP055344482, US ISSN: 0897-7151, DOI: 10.1089/neu.2010.1632
- JAMES J DONKIN ET AL: "Substance P is Associated with the Development of Brain Edema and Functional Deficits after Traumatic Brain Injury", JOURNAL OF CEREBRAL BLOOD FLOW & METABOLISM, vol. 29, no. 8, 1 August 2009 (2009-08-01) , pages 1388-1398, XP055344477, US ISSN: 0271-678X, DOI: 10.1038/jcbfm.2009.63
- ROBERT A STERN ET AL: "Long-term Consequences of Repetitive Brain Trauma: Chronic Traumatic Encephalopathy", PM&R, vol. 3, no. 10, 26 October 2011 (2011-10-26), pages S460-S467, XP028328976, ISSN: 1934-1482, DOI: 10.1016/J.PMRJ.2011.08.008 [retrieved on 2011-08-19]
- ANN C. MCKEE ET AL: "Chronic Traumatic Encephalopathy in Athletes", JOURNAL OF NEUROPATHOLOGY AND EXPERIMENTAL NEUROLOGY, vol. 68, no. 7, 1 July 2009 (2009-07-01), pages 709-735, XP055174514, ISSN: 0022-3069, DOI: 10.1097/NEN.0b013e3181a9d503

## Description

### Field of the Invention

The present invention relates to a method of preventing and/or treating chronic traumatic encephalopathy.

### Background of the Invention

Concussion has become an important public health problem in the United States, Australia and elsewhere internationally. It is common in a number of contact sports including the Australian football codes such as AFL and NRL, ice hockey, American football, and boxing, amongst others. In the United States alone, over 300,000 sports related concussions occur annually and numbers are increasing worldwide (Ellenbogen et al., 2010, World Neurosurg. 74, 560-575). Concussive injuries are also a problem in the military and industrial worksites. In the case of the former traumatic brain injury resulting from exposure to the force of a detonation trigger similar neuropathological mechanisms leading to neuropathology and sequelae indistinguishable to chronic traumatic encephalopathy (Goldstein et al (2012) Sci. Transl. Med. 4(134): 1-16). Concussion causes no gross pathology, such as hemorrhage, and no abnormalities on structural brain imaging (McCrory et al., 2009, Phys. Sportsmed. 37, 141-159). There also may be no loss of consciousness, but many other complaints such as dizziness, nausea, reduced attention and concentration, memory problems, and headache have been reported. A greater likelihood of unconsciousness occurs with more severe concussions. These types of concussive head impacts are very frequent in American football whose athletes, especially linemen and linebackers, may be exposed to more than 1,000 impacts per season (Crisco et al., 2010, J. Athl. Train. 45, 549-559). The effects of multiple concussions are becoming better recognized in these professional footballers, but much less is known about the long term-effects of repeated concussion in the brains of amateur teenagers and adolescents. Moreover, the amateur codes of football are less regulated than the professional codes, and the adolescent brain may be more vulnerable to concussion. The better-developed neck musculature of the professional footballer, the more strictly controlled tackling and the better aftercare of the concussed professional means that the long-term public health problem of concussion in sport is grossly underestimated.

Military personnel who have experienced concussion experience a range of detrimental and chronic medical conditions. Concussion occurring among soldiers deployed in Iraq is strongly associated with PTSD and physical health problems 3 to 4 months after the soldiers return home. PTSD and depression are important mediators of the relationship between mild traumatic brain injury and physical health problems. PTSD was strongly associated with mild traumatic brain injury. It was reported that overall, 43.9% of soldiers who reported loss of consciousness met the criteria for PTSD, as compared with 27.3% of those with altered mental status, 16.2% of those with other injuries, and 9.1% of those with no injuries (Hoge et al, N Engl J Med. 2008; 358,453-63). Also, more than 1 in 3 returning military troops who have sustained a deployment-related concussion have headaches that meet criteria for posttraumatic headache (Theeler et al., 2010, Headache: J Head and Face Pain 50, 1262-1272). It has been shown that nearly 15% of combat personnel sustained concussion whilst on duty (Hoge et al, N Engl J Med. 2008; 358,453-63). Repeated concussion is a serious issue for combat personnel, with a study showing that a majority of concussion incidents were blast related. The median time between events was 40 days, with 20% experiencing a second event within 2 weeks of the first and 87% within 3 months (MacGregor et al, 2011, J Rehab Research and Develop, 48, 1269-1278). The impact of concussion and PTSD has resulted in a significant economic burden, (The Congress of the United States - Congressional Budget Office, The Veterans Health Administration's Treatment of PTSD and Traumatic Brain Injury Among Recent Combat Veterans, February 2012). While an isolated concussion has been widely considered to be an innocuous event, recent studies (McKee et al., 2009, J Neuropath Exp Neurol 68, 709-735; Blennow et al., 2012, Neuron 76, 886-99) have suggested that repeated concussion is associated with the development of a neurodegenerative disorder known as chronic traumatic encephalopathy (CTE). CTE is regarded as a disorder that often occurs in midlife, years or decades after the sports or military career has ended (McKee et al., 2009, J Neuropath Exp Neurol 68, 709-735; Stern et al., 2011, Physical Med. Rehab. 3, S460-7). About one-third of CTE cases are progressive, but clinical progression is not always sequential or predictable. The clinical symptoms vary extensively, which is probably due to varying, multiple damage sites amongst athletes with the condition (Stern et al., 2011, Physical Med. Rehab. 3, S460-7). The severity varies from mild complaints to severe deficits accompanied by dementia, Parkinson-like symptoms, and behavioral changes. Clinical symptoms include neurological and cognitive complaints together with psychiatric and behavioral disturbances. Early neurological symptoms may include speech problems and impaired balance, while later symptoms include ataxia, spasticity, impaired coordination, and extrapyramidal symptoms, with slowness of movements and tremor (Blennow et al., 2012, Neuron 76, 886-99; Stern et al., 2011, Physical Med. Rehab. 3, S460-7). Cognitive problems, such as attention deficits and memory disturbances, often become major factors in later stages of the disease, although may occur at varying times throughout the course of CTE. Psychiatric and behavioral problems include lack of insight and judgment, depression, disinhibition and euphoria, hypomania, irritability, aggressiveness and suicidal tendencies.

In post-mortem studies of athletes with CTE, the extensive presence of neurofibrillary tangles has been reported (McKee et al., 2009, J Neuropath Exp Neurol 68, 709-735; Stern et al., 2011, Physical Med. Rehab. 3, S460-7). Tangles are found intracellularly in the cytoplasm of neurons and consist of threadlike aggregates of hyperphosphorylated tau protein. Tau is a normal axonal protein that binds to microtubules via their microtubule binding domains, thus promoting microtubule assembly and stability. The hyperphosphorylated form of tau causes disassembly of microtubules and thus impaired axonal transport, leading to compromised neuronal and synaptic function, increased propensity of tau aggregation, and subsequent formation of insoluble fibrils and tangles. Unlike in Alzheimer's disease, tangles in athletes with CTE tend to accumulate perivascularly within the superficial neocortical layers, particularly at the base of the sulci. Tau pathology in CTE is also patchy and irregularly distributed, possibly related to the many different directions of mechanical force induced by physical trauma (McKee et al., 2009, J Neuropath Exp Neurol 68, 709-735). It is the accumulation of hyperphosphorylated tau protein that is thought to result in the development of CTE and its associated psychiatric and behavioral disturbances.

Given these psychiatric and behavioural disturbances in athletes with CTE, there is a clear need for a therapeutic intervention to prevent and/or treat chronic traumatic encephalopathy.

A reference herein to a patent document or other matter which is given as prior art is not to be taken as an admission that that document or matter was known or that the information it contains was part of the common general knowledge as at the priority date of any of the claims.

### Summary of the Invention

The present invention relates to treating conditions associated with chronic traumatic encephalopathy or a related condition having overlapping neuropathology and sequelae after concussive injury.

Accordingly, in one aspect the present invention provides a compound of Formula (I), or a pharmaceutically acceptable salt, or solvate thereof: 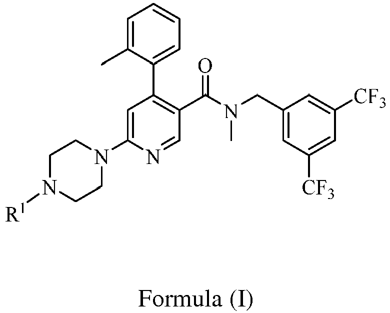 wherein R¹ is H or C₁₋₄ alkyl, or a pharmaceutically acceptable salt, or solvate thereof, for use for preventing and/or treating chronic traumatic encephalopathy (CTE) in a subject.

The present disclosure also discloses a method of preventing and/or treating chronic traumatic encephalopathy or a related condition in a subject, the method including administering to the subject an effective amount of a compound of formula (I): 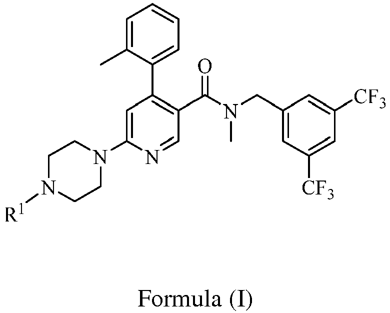 wherein R¹ is H or C₁₋₄ alkyl, or a pharmaceutically acceptable salt, solvate, or prodrug thereof.

In another aspect, the present disclosure also discloses use of a compound of formula (I), or a pharmaceutically acceptable salt, solvate, or prodrug thereof, in the preparation of a medicament for preventing and/or treating chronic traumatic encephalopathy or a related condition in a subject.

In a further aspect the present disclosure also discloses a pharmaceutical composition when used to treat chronic traumatic encephalopathy or a related condition, the composition including a compound of formula (I), or a pharmaceutically acceptable salt, solvate, or prodrug thereof.

In still a further aspect the present disclosure also discloses a method of inhibiting progression of a disease, condition or state associated with tau hyperphosphorylation in a subject, the method including administering to the subject an effective amount of a compound of formula (I), or a pharmaceutically acceptable salt, solvate, or prodrug thereof.

In still a further aspect the present disclosure also discloses use of a compound of formula (I), or a pharmaceutically acceptable salt, solvate, or prodrug thereof, in the preparation of a medicament for inhibiting progression of a disease, condition or state associated with tau hyperphosphorylation in a subject, for instance a concussive injury.

In a further aspect the disclosure discloses a method for treating a subject with a concussive injury, including the step of administering to said subject an effective amount of a compound of formula (I), or a pharmaceutically acceptable salt, solvate, or prodrug thereof.

In a further aspect the disclosure discloses methods for treating psychiatric and behavioural problems associated with CTE in a subject in need thereof, including the step of administering to said subject an effective amount of a compound of formula (I), or a pharmaceutically acceptable salt, solvate, or prodrug thereof.

In an example the psychiatric and behavioural problems are selected from the group consisting of depression, irritability, disinhibition and euphoria, hypomania, aggressiveness and suicidal tendencies.

In a further aspect the disclosure discloses methods for treating cognitive problems associated with CTE, in a subject in need thereof, including the step of administering to said subject an effective amount of a compound of formula (I), or a pharmaceutically acceptable salt, solvate, or prodrug thereof.

In an example the cognitive problems associated with CTE are selected from the group consisting of attention deficits and memory distrubances.

Various terms that will be used throughout the specification have meanings that will be well understood by a skilled addressee. However, for ease of reference, some of these terms will now be defined.

The term "chronic traumatic encephalopathy (CTE)" as used throughout the specification is a condition appearing in response to repeated concussion resulting in accumulation of neurofibrillary tangles consisting of hyperphosphorylated tau protein. The perivascular appearance of these neurofibrillary tangles within the superficial neocortical layers, and particularly at the base of the sulci, is unique to athletes and has been associated with the subsequent development of psychiatric and behavioural disturbances.

The term "tau hyperphosphorylation" as used throughout the specification is to be understood to mean the phosphorylated form of tau that causes disassembly of microtubules and thus impaired axonal transport, leading to compromised neuronal and synaptic function, increased propensity of tau aggregation, and subsequent formation of insoluble fibrils and tangles.

In this regard, a disease condition or state known as chronic traumatic encephalopathy is associated with accumulation of hyperphosphorylated tau protein, leading to compromised neuronal and synaptic function, increased propensity of tau aggregation, subsequent formation of insoluble fibrils and tangles, and the development of psychiatric and behavioural disturbances.

A related condition is a condition having overlapping neuropathology and sequelae.

The term "prevent" as used throughout the specification is to be understood to mean an intervention that prevents or delays the onset of a disease, condition or state in a subject. The term "treat" as used throughout the specification is to be understood to mean an intervention that improves the prognosis and/or state of a subject with respect to a disease, condition or state.

The term "subject" as used throughout the specification is to be understood to mean a human or animal subject.

The present disclosure furthermore discloses military applications such as administering a compound of formula (I), or a pharmaceutically acceptable salt, solvate, or prodrug thereof, at an aid station shortly after a blast injury or traumatic events involving the head or during post recovery.

It will also be understood that the present invention further includes within its scope veterinary applications. For example, the animal subject may be a mammal, a primate, a livestock animal (eg. a horse, a cow, a sheep, a pig, or a goat), a companion animal (eg. a dog, a cat), a laboratory test animal (eg. a mouse, a rat, a guinea pig, a bird, a rabbit), an animal of veterinary significance, or an animal of economic significance.

### Brief Description of the Figures

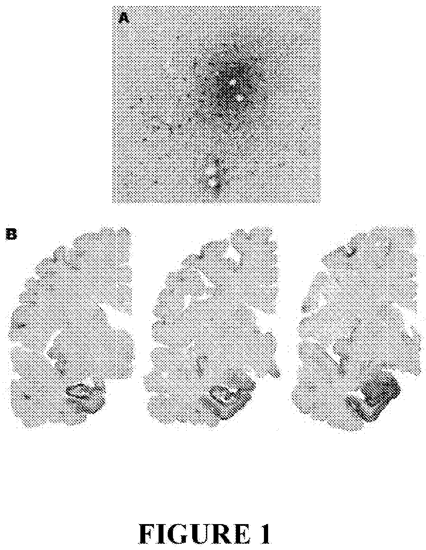

Figure 1 shows immunohistology using antibody for phosphorylated tau of sections of a human brain diagnosed with CTE demonstrating the perivascular appearance (A) of hyperphosphorylated tau within the superficial neocortical layers, and particularly at the base of the sulci (B) (from McKee et al, 2009, J Neuropath Exp Neurol 68, 709-735).

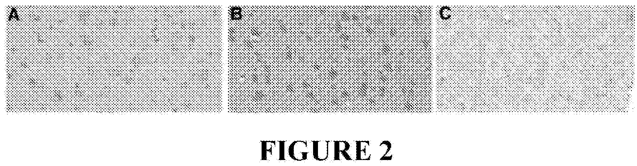

Figure 2 shows the effects of a compound of formula (I") on tau phosphorylation after concussive injury. Note that concussive injury in the rat causes extensive tau phosphorylation (B) by 3 days after the concussive event compared to non-injured animals (A). The administration of a compound of formula (I") at 30 minutes after the induction of injury results in almost complete inhibition of tau phosphorylation at this 3 day time point (C).

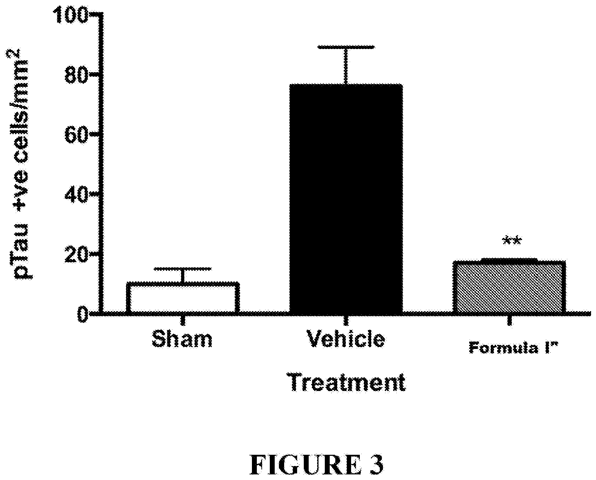

Figure 3 shows objective assessment of the immunolabelling seen in Figure 2 above as achieved through colour deconvolution techniques to reveal the percentage of DAB in the scanned slides as previously described in detail (Helps et. al., Appl Immunohistochem Mol Morphol 20(1): 82-90).

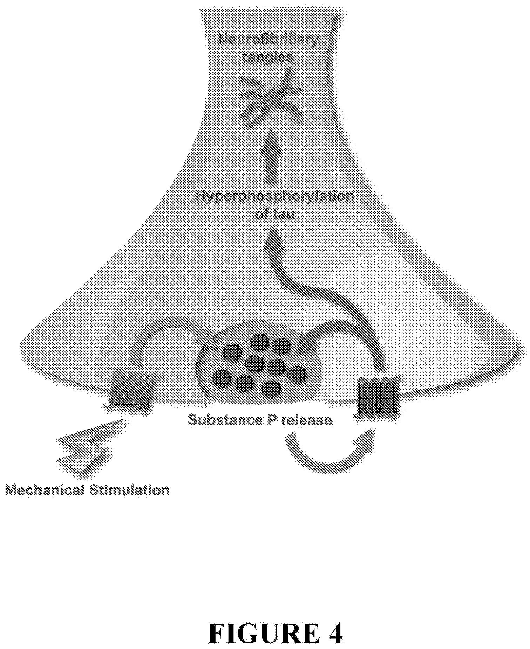

Figure 4 shows a schematic model of how concussive events result in substance P release and subsequent hyperphosphorylation of tau. Neuronal sensory fibres surrounding blood vessels undergo stretch in response to a concussive event. The resultant mechanical stimulation activates mechanoreceptors and triggers substance P release. Substance P binds to its receptors, activating an array of kinases known to be associated with hyperphosphorylation of tau. Hyperphosphorylation of tau destabilises microtubules and results in neurofibrillary tangles.

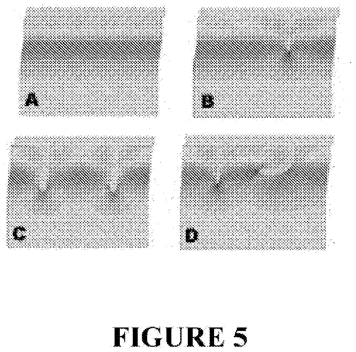

Figure 5 shows stress fields following simulated rotational acceleration in models replicating brain tissue with no sulci (A) to brain tissue with complex sulci formation (B-D). Higher stress is indicated as black and is focused at the base of the sulci irrespective of the sulcus morphology.

### General Description of the Invention

As described above, the present disclosure discloses a method of preventing and/or treating chronic traumatic encephalopathy or a related condition in a subject, the method including administering to the subject an effective amount of a compound of formula (I), or a pharmaceutically acceptable salt, solvate, or prodrug thereof.

In one embodiment the compound of formula (I) is represented by

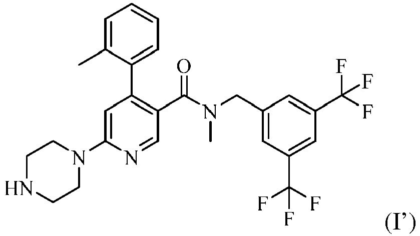

In another embodiment the compound of formula (I) is represented by

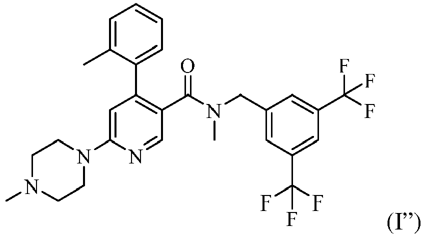

Compounds of formula (I) may be prepared as described in EP 1 103 546 B1, US 2003/083345 A discloses the compounds of formula (I) for use in the prevention and/or treatment of traumatic brain, spinal or nerve injury.

This example of the present disclosure is directed to preventing and/or treating a disease, condition or state associated with tau hyperphosphorylation by administering to a subject one or more compounds of formula (I), or a pharmaceutically acceptable salt, solvate, or prodrug thereof.

Tau hyperphosphorylation may be induced by a variety of reasons, including for example, a concussive event or a mechanical impact that activates brain mechanoreceptors. In this regard, tau hyperphosphorylation may be associated, for example, with either or both an accumulation of hyperphosphorylated tau over time as measured within the one subject, or may be an accumulation of hyperphosphorylated tau in one subject compared to the accumulation of hyperphosphorylated tau in a population.

Diseases, conditions or states associated with accumulation of hyperphosphorylated tau in a subject in the various examples of the present disclosure include chronic traumatic encephalopathy (CTE).

Chronic traumatic encephalopathy (CTE) is normally classified as a disease associated with accumulation of tangles containing hyperphosphorylated tau, with these tangles tending to accumulate perivascularly within the superficial neocortical layers, particularly at the base of the sulci. There is currently no blood or laboratory test that is definitive for the diagnosis of CTE, with disease confirmation usually occurring after postmoretm examination of brain tissue. Nonetheless, a number of clinical criteria plus a history of concussive events in the subject are usually sufficient in making a tentative diagnosis. In this regard, the Diagnostic and Statistical Manual of Mental Disorders (American Psychiatric Association) is commonly used to assess a number of parameters to provide an indication of the presence and severity of CTE in a subject. Nuclear medical imaging, including Positron Emission Tomography (PET), may also be used to assess the presence and severity of CTE. Methods of assessing CTE in a subject using PET include for example Small et al. (2013) Am. J. Geriatr. Psychiatry. 21: 138-144.

Accordingly, in a further example the disclosure may include a CTE diagnostic step which may be performed by injecting the subject with a PET molecular imaging probe (to visualise CTE in living humans). Such imaging probes are known, for instance, FDDNP(2-1-{6-[(2-[F-18]fluoroethyl)(methyl)amino]-2-napthyl}ethylidene)malononitrite. Such probes are able to visualise tau tangles.

In a further example the diagnostic step may include an assessment of the plasma levels of total tau (T-tau) using an immunoassay for instance, as described in Rissen et al, Nature Biotechnology 2010;28:595-599.

In an example diagnosis of CTE may be made based on a plasma level of Tau (based on the aforementioned assay) of above 1.5 ngL⁻¹, for instance, above 1.6, above 1.7, above 1.8, above 1.9, above 2.0, above 2.1, above 2.2, above 2.3, above 2.4, above 2.5, above 2.6, or above 2.7 ngL⁻¹.

In one specific embodiment, the disease, condition or state associated with accumulation of hyperphosphorylated tau is chronic traumatic encephalopathy.

In another specific example, the diverse, condition or state associated with accumulation of hyperphosphorylated tau is a concussive event or injury.

One or more compounds of formula (I), or a pharmaceutically acceptable salt, solvate, or prodrug thereof, may also be used in the preparation of a medicament for preventing and/or treating chronic traumatic encephalopathy or a related condition.

Accordingly, in another example the present disclosure discloses use of a compound of formula (I), or a pharmaceutically acceptable salt, solvate, or prodrug thereof, in the preparation of a medicament for preventing and/or treating chronic traumatic encephalopathy or a related condition.

One or more compounds of formula (I), or a pharmaceutically acceptable salt, solvate, or prodrug thereof, may also be used in a pharmaceutical composition, to prevent and/or treat chronic traumatic encephalopathy or a related condition.

Accordingly, in another example the present disclosure discloses a pharmaceutical composition when used to prevent and/or treat chronic traumatic encephalopathy or a related condition, the composition including a compound of formula (I), or a pharmaceutically acceptable salt, solvate, or prodrug thereof.

The administration of one or more compounds of formula (I), or a pharmaceutically acceptable salt, solvate, or prodrug thereof, may also be used to inhibit progression of the disease, condition or state associated with chronic traumatic encephalopathy or a related condition in the subject.

Accordingly, in another example the present disclosure discloses a method of inhibiting progression of chronic traumatic encephalopathy or a related condition, the method including administering to the subject an effective amount of a compound of formula (I), or a pharmaceutically acceptable salt, solvate, or prodrug thereof.

The effective amount of a compound of formula (I), or a pharmaceutically acceptable salt, solvate, or prodrug thereof, to be delivered in the various embodiments of the present invention is not particularly limited, so long as it is within such an amount and in such a form that generally exhibits a useful or therapeutic effect. The term "effective amount" is the quantity which when delivered, improves the prognosis of the subject. The amount to be delivered will depend on the particular characteristics of the condition being treated, the mode of delivery, and the characteristics of the subject, such as general health, other diseases, age, sex, genotype, body weight and tolerance to drugs.

In an example, an effective amount of a compound of formula (I), or a pharmaceutically acceptable salt, solvate, or prodrug thereof, is an amount to be delivered to restore plasma levels of total tau (T-tau) (for instance by the immunoassay identified hereinbefore) to less than 1 ngL⁻¹, for instance, less than 0.9 ngL⁻¹, or less than 0.8 ngL⁻¹. This may involve a single dose or repeated dosages.

Accordingly, a suitable dosage of the compound of formula (I), or a pharmaceutically acceptable salt, solvate, or prodrug thereof, for delivery to the desired site of action in the various examples of the present disclosure may be selected.

In an example the method relates to a method for treating a concussive injury which involves a patient being exposed to multiple (more than one) concussive events. In such a method, the attendant physician would determine that the subject is concussed and that the subject has had at least one previous concussion. Methods for determining whether or nor a subject has been concussed includes for instance a variety of neuropsychological assessment tools (Kelly et al., 2012, Arch Clin Neuropsycho 27, 375-88; Echemendia et al., 2012, Clin Neuropsychol 26, 1077-91). However, the detection of loss of memory, an alteration of mental state (mental cloudiness, headache, dizziness, confusion, disorientation), possible loss of consciousness, or focal neurological deficits is more commonly used for on-field diagnosis of a concussive event. Other diagnostic criteria are outlined in detail in the American Society for Sports Medicine position statement (Br J Sports Med, 2013, 47, 15-26) and are summarised on the regularly updated Centres for Disease Control and Prevention (USA) website (http://www.cdc.gov/concussion/ sports/index.html). Once this has been established the physician would then administered an effective amount of a compound of formula (I), or a pharmaceutically acceptable salt, solvate, or prodrug thereof.

In one example, the dosage of the compound of formula (I), or a pharmaceutically acceptable salt, solvate, or prodrug thereof, administered to a subject in the various embodiments of the present is in the range from 0.1 mg/kg to 100 mg/kg. For instance, the dosage amount may be 0.5 mg/kg, 1.0 mg/kg, 2.0 mg/kg, 3.0 mg/kg, 4.0 mg/kg, 5.0 mg/kg, 6.0 mg/kg, 7.0 mg/kg, 8.0 mg/kg, 9.0 mg/kg, 10.0 mg/kg, 11.0 mg/kg, 12.0 mg/kg, 13.0 mg/kg, 14.0 mg/kg, 15.0 mg/kg, 16.0 mg/kg, 17.0 mg/kg, 18.0 mg/kg, 19.0 mg/kg, 20.0 mg/kg, 21.0 mg/kg, 22.0 mg/kg, 23.0 mg/kg, 24.0 mg/kg, 25.0 mg/kg, 26.0 mg/kg, 27.0 mg/kg, 28.0 mg/kg, 29.0 mg/kg, 30.0 mg/kg, 31.0 mg/kg, 32.0 mg/kg, 33.0 mg/kg, 34.0 mg/kg, 35.0 mg/kg, 36.0 mg/kg, 37.0 mg/kg, 38.0 mg/kg, 39.0 mg/kg, 40.0 mg/kg, 41.0 mg/kg, 42.0 mg/kg, 43.0 mg/kg, 44.0 mg/kg, 45.0 mg/kg, 46.0 mg/kg, 47.0 mg/kg, 48.0 mg/kg, 49.0 mg/kg, 50.0 mg/kg, 51.0 mg/kg, 52.0 mg/kg, 53.0 mg/kg, 54.0 mg/kg, 55.0 mg/kg, 56.0 mg/kg, 57.0 mg/kg, 58.0 mg/kg, 59.0 mg/kg, 60.0 mg/kg, 61.0 mg/kg, 62.0 mg/kg, 63.0 mg/kg, 64.0 mg/kg, 65.0 mg/kg, 66.0 mg/kg, 67.0 mg/kg, 68.0 mg/kg, 69.0 mg/kg, 70.0 mg/kg, 71.0 mg/kg, 72.0 mg/kg, 73.0 mg/kg, 74.0 mg/kg, 75.0 mg/kg, 76.0 mg/kg, 77.0 mg/kg, 78.0 mg/kg, 79.0 mg/kg, 80.0 mg/kg, 81.0 mg/kg, 82.0 mg/kg, 83.0 mg/kg, 84.0 mg/kg, 85.0 mg/kg, 86.0 mg/kg, 87.0 mg/kg, 88.0 mg/kg, 89.0 mg/kg, 90.0 mg/kg, 91.0 mg/kg, 92.0 mg/kg, 93.0 mg/kg, 94.0 mg/kg, 95.0 mg/kg, 96.0 mg/kg, 97.0 mg/kg, 98.0 mg/kg, or 99.0 mg/kg.

In a specific example, the compound of formula (I), or a pharmaceutically acceptable salt, solvate, or prodrug thereof, is administered to the subject at a dose of 0.25 mg/kg to 25 mg/kg.

In a further example, the compound of formula (I), or a pharmaceutically acceptable salt, solvate, or prodrug thereof, is administered to the subject at a dose of 1 mg/kg to 10 mg/kg within 24 hours of the concussive event such that the plasma levels of total tau (T-Tau) is less than about 1 ngL⁻¹ after 7 days.

In an example the compound shall be administered as a prophylaxis for injury associated with concussion post the injury event.

In an example the compound shall be administered as a treatment for injury associated with concussion post the injury event.

In an embodiment the compound shall be administered as a prophylaxis to reduce hyperphosphorylated Tau.

In an embodiment the compound shall be administered as a treatment to reduce hyperphosphorylated Tau.

In an example the effective amount is an amount which is able to maintain the blood concentration of the compound of formula (I), or a pharmaceutically acceptable salt, or solvate, thereof, in the therapeutic range for at least 3 days, for instance at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, or at least 20 days.

In an example the effective amount is administered as a single or multiple dose.

In an example the effective amount is administered as a single or multiple oral dose.

Accordingly, in another aspect the disclosure discloses a method for treating a subject which has been exposed to multiple concussive events including the step of administering to the subject a compound of formula (I), or a pharmaceutically acceptable salt, solvate, or prodrug thereof, as a single oral dose in an amount which is able to maintain the blood concentration of the compound of formula (I), or a pharmaceutically acceptable salt, solvate, or prodrug thereof, in the therapeutic range for at least 3 days, wherein the administration step is performed after the second concussive event and again after each additional concussive event as required.

In an example the compound of formula (I), or a pharmaceutically acceptable salt, solvate, or prodrug thereof, is administered within 24 hours of the concussive event.

In an example administration is provided within 20 hours such as within, 19 hours, 18 hours, 17 hours, 16 hours, 15 hours, 14 hours, 13 hours, 12 hours, 11 hours, 10 hours, 9 hours, 8 hours, 7 hours, 6 hours, 5 hours, 4 hours, 3 hours, 2 hours and within 1 hour, of the concussive event.

In an example the oral dose is in the form of a tablet, capsule, drink solutions or parenteral.

Generally, the dosage of the compound of formula (I), or a pharmaceutically acceptable salt, solvate, or prodrug thereof, in a pharmaceutical composition may be in the range from 10-5,000 mg per subject, and typically will be in the range of 50-2,000 mg per subject.

Methods for the preparation of pharmaceutical compositions are known in the art, for example as described in Remington's Pharmaceutical Sciences, 18th ed., 1990, Mack Publishing Co., Easton, Pa. and U.S. Pharmacopeia: National Formulary, 1984, Mack Publishing Company, Easton, Pa.

As discussed previously herein, administration and delivery of the compositions may be for example by the intravenous, intraperitoneal, subcutaneous, intramuscular, oral, or topical route, or by direct injection. The mode and route of administration in most cases will depend on the severity and frequency of the concussive events.

The dosage form, frequency and will depend on the mode and route of administration.

As described above, the administration of the compound of formula (I), or a pharmaceutically acceptable salt, solvate, or prodrug thereof, may also include the use of one or more pharmaceutically acceptable additives, including pharmaceutically acceptable salts, amino acids, polypeptides, polymers, solvents, buffers, excipients, preservatives and bulking agents, taking into consideration the particular physical, microbiological and chemical characteristics of the agents to be administered.

For example, the compound of formula (I), or a pharmaceutically acceptable salt, solvate, or prodrug thereof, can be prepared into a variety of pharmaceutically acceptable compositions in the form of, e.g., an aqueous solution, an oily preparation, a fatty emulsion, an emulsion, a lyophilised powder for reconstitution, etc. and can be administered as a sterile and pyrogen free intramuscular or subcutaneous injection or as injection to an organ, or as an embedded preparation or as a transmucosal preparation through nasal cavity, rectum, uterus, vagina, lung, etc. The composition may be administered in the form of oral preparations (for example solid preparations such as tablets, caplets, capsules, granules or powders; liquid preparations such as syrup, emulsions, dispersions or suspensions).

The term "pro-drug" is used in its broadest sense and encompasses those derivatives that are converted *in vivo* to the compounds of the invention. Such derivatives would readily occur to those skilled in the art.

Compositions containing the compound of formula (I), or a pharmaceutically acceptable salt, solvate, or prodrug thereof, may also contain one or more pharmaceutically acceptable preservatives, buffering agents, diluents, stabilisers, chelating agents, viscosity enhancing agents, dispersing agents, pH controllers, or isotonic agents.

Examples of suitable preservatives are benzoic acid esters of para-hydroxybenzoic acid, propylene glycol, phenols, phenylethyl alchohol or benzyl alcohol. Examples of suitable buffers are sodium phosphate salts, citric acid, tartaric acid and the like. Examples of suitable stabilisers are, antioxidants such as alpha-tocopherol acetate, alpha-thioglycerin, sodium metabisulphite, ascorbic acid, acetylcysteine, 8-hydroxyquinoline, chelating agents such as disodium edetate. Examples of suitable viscosity enhancing agents, suspending or dispersing agents are substituted cellulose ethers, substituted cellulose esters, polyvinyl alchohol, polyvinylpyrrolidone, polyethylene glcols, carbomer, polyoxypropylene glycols, sorbitan monooleate, sorbitan sesquioleate, polyoxyethylene hydrogenated castor oil 60.

Examples of suitable pH controllers include hydrochloric acid, sodium hydroxide and the like. Examples of suitable isotonic agents are glucose, D-sorbitol or D-mannitol, sodium chloride.

The administration of a compound of formula (I), or a pharmaceutically acceptable salt, solvate, or prodrug thereof, in the various examples of the present disclosure may also be in the form of a composition containing a pharmaceutically acceptable carrier, diluent, excipient, suspending agent, lubricating agent, adjuvant, vehicle, delivery system, emulsifier, disintegrant, absorbent, preservative, surfactant, colorant, glidant, anti-adherant, binder, flavorant or sweetener, taking into account the physical, chemical and microbiological properties of the agents being administered.

For these purposes, the composition may be administered orally, parenterally, by inhalation spray, adsorption, absorption, topically, rectally, nasally, mucosally, transdermally, bucally, vaginally, intraventricularly, via an implanted reservoir in dosage formulations containing conventional non-toxic pharmaceutically-acceptable carriers, or by any other convenient dosage form. The term parenteral as used herein includes subcutaneous, intravenous, intramuscular, intraperitoneal, intrathecal, intraventricular, intrasternal, and intracranial injection or infusion techniques.

When administered parenterally, the compositions will normally be in a unit dosage, sterile, pyrogen free injectable form (solution, suspension or emulsion, which may have been reconstituted prior to use), which is generally isotonic with the blood of the recipient with a pharmaceutically acceptable carrier. Examples of such sterile injectable forms are sterile injectable aqueous or oleaginous suspensions. These suspensions may be formulated according to techniques known in the art using suitable vehicles, dispersing or wetting agents and suspending agents. The sterile injectable forms may also be sterile injectable solutions or suspensions in non-toxic parenterally acceptable diluents or solvents, for example, as solutions in 1,3-butanediol. Among the pharmaceutically acceptable vehicles and solvents that may be employed are water, ethanol, glycerol, saline, Ringer's solution, dextrose solution, isotonic sodium chloride solution, and Hanks' solution. In addition, sterile, fixed oils are conventionally employed as solvents or suspending mediums. For this purpose, any bland fixed oil may be employed including synthetic mono- or di-glycerides, corn, cottonseed, peanut, and sesame oil. Fatty acids such as ethyl oleate, isopropyl myristate, and oleic acid and its glyceride derivatives, including olive oil and castor oil, especially in their polyoxyethylated versions, are useful in the preparation of injectables. These oil solutions or suspensions may also contain long-chain alcohol diluents or dispersants.

The carrier may contain minor amounts of additives, such as substances that enhance solubility, isotonicity, and chemical stability, for example anti-oxidants, buffers and preservatives.

In addition, the compositions may be in a form to be reconstituted prior to administration. Examples include lyophilisation, spray drying and the like to produce a suitable solid form for reconstitution with a pharmaceutically acceptable solvent prior to administration.

Compositions may include one or more buffers, bulking agents, isotonic agents and cryoprotectants and lyoprotectants. Examples of excipients include, phosphate salts, citric acid, non-reducing such as sucrose or trehalose, polyhydroxy alcohols, amino acids, methylamines, and lyotropic salts which are usually used instead of reducing sugars such as maltose or lactose.

When administered orally, the compound of formula (I), or a pharmaceutically acceptable salt, solvate, or prodrug thereof, will usually be formulated into unit dosage forms such as tablets, caplets, cachets, powder, granules, beads, chewable lozenges, capsules, liquids, aqueous suspensions or solutions, or similar dosage forms, using conventional equipment and techniques known in the art. Such formulations typically include a solid, semisolid, or liquid carrier. Exemplary carriers include excipients such as lactose, dextrose, sucrose, sorbitol, mannitol, starches, gum acacia, calcium phosphate, mineral oil, cocoa butter, oil of theobroma, alginates, tragacanth, gelatin, syrup, substituted cellulose ethers, polyoxyethylene sorbitan monolaurate, methyl hydroxybenzoate, propyl hydroxybenzoate, talc, magnesium stearate, and the like.

A tablet may be made by compressing or molding the agent optionally with one or more accessory ingredients. Compressed tablets may be prepared by compressing, in a suitable machine, the active ingredient in a free-flowing form such as a powder or granules, optionally mixed with a binder, lubricant, inert diluent, surface active, or dispersing agent. Moulded tablets may be made by moulding in a suitable machine, a mixture of the powdered active ingredient and a suitable carrier moistened with an inert liquid diluent.

The administration of the compound of formula (I), or a pharmaceutically acceptable salt, solvate, or prodrug thereof, may also utilize controlled release technology.

The compound of formula (I), or a pharmaceutically acceptable salt, solvate, or prodrug thereof, may also be administered as a sustained-release pharmaceutical composition. To further increase the sustained release effect, the agent may be formulated with additional components such as vegetable oil (for example soybean oil, sesame oil, camellia oil, castor oil, peanut oil, rape seed oil); middle fatty acid triglycerides; fatty acid esters such as ethyl oleate; polysiloxane derivatives; alternatively, water-soluble high molecular weight compounds such as hyaluronic acid or salts thereof, carboxymethylcellulose sodium hydroxypropylcellulose ether, collagen polyethylene glycol polyethylene oxide, hydroxypropylmethylcellulosemethylcellulose, polyvinyl alcohol, polyvinylpyrrolidone.

Alternatively, the compound of formula (I), or a pharmaceutically acceptable salt, solvate, or prodrug thereof, may be incorporated into a hydrophobic polymer matrix for controlled release over a period of days. The agent may then be moulded into a solid implant, or externally applied patch, suitable for providing efficacious concentrations of the agents over a prolonged period of time without the need for frequent re-dosing. Such controlled release films are well known to the art. Other examples of polymers commonly employed for this purpose that may be used include nondegradable ethylene-vinyl acetate copolymer a degradable lactic acid-glycolic acid copolymers, which may be used externally or internally. Certain hydrogels such as poly(hydroxyethylmethacrylate) or poly(vinylalcohol) also may be useful, but for shorter release cycles than the other polymer release systems, such as those mentioned above.

The carrier may also be a solid biodegradable polymer or mixture of biodegradable polymers with appropriate time-release characteristics and release kinetics. The agent may then be moulded into a solid implant suitable for providing efficacious concentrations of the agents over a prolonged period of time without the need for frequent re-dosing. The agent can be incorporated into the biodegradable polymer or polymer mixture in any suitable manner known to one of ordinary skill in the art and may form a homogeneous matrix with the biodegradable polymer, or may be encapsulated in some way within the polymer, or may be moulded into a solid implant.

For topical administration, the compound of formula (I), or a pharmaceutically acceptable salt, solvate, or prodrug thereof, may be in the form of a solution, spray, lotion, cream (for example a non-ionic cream), gel, paste or ointment. Alternatively, the composition may be delivered via a liposome, nanosome, rivosome, or nutri-diffuser vehicle.

It will be appreciated that other forms of administration of agents are also contemplated, including the use of a nucleic acid encoding a polypeptide for delivering of such agents.

### Description of Specific Examples

Reference will now be made to experiments that embody the above general principles of the present invention.

### Example 1

### Concussion results in accumulation of hyperphosphorylated tau.

A number of clinical and experimental studies have now shown that there is an accumulation of hyperphosphorylated tau following concussive injury. Accumulation of neurofibrillary tangles containing hyperphosphorylated tau is a hallmark pathology of chronic traumatic encephalopathy, especially when this accumulation is perivascular and predominantly found within the superficial neocortical layers, particularly at the base of the sulci. In human studies (McKee et al., 2009, J Neuropath Exp Neurol 68, 709-735) such a distribution of hyperphosphorylated tau is readily apparent in subjects who have a history of repeated concussive events (Figure 1). In this particular example, localization of hyperphosphorylated tau is shown in an NFL football player with a history of repeated concussion. Note the perivascular localization of hyperphosphorylated tau (A) with highest accumulations at the base of the sulci. This pathology is unique to chronic traumatic encephalopathy. Similar accumulations of hyperphosphorylated tau have been shown following experimental concussion in animals, although the absence of sulci in the experimental animals used in these studies to date has precluded the demonstration that such accumulation replicates the human pattern of localisation at the base of the sulci.

### Example 2

### Concussion results in perivascular substance P release

Having established that hyperphosphorylated tau accumulates perivascularly following concussive injury, we used an animal model of concussion to investigate whether concussion causes perivascular release of substance P. We developed a rodent model of concussion to replicate the concussive event (Donkin et al., 2004, 7th International Neurotrauma Symposium, pp 75-78, Medimond Publishers, Bologna, Italy) and subsequently determined whether substance P was released after such an event. There was a clear increase in brain perivascular substance P immunoreactivity after the concussive event (Figure 2). We propose that mechanical stimulation of sensory nerve fibres was responsible for this perivascular release of substance P. These results are consistent with previous studies in non-brain tissue demonstrating that mechanical stimulation of sensory nerve fibres induces substance P release (Ang et al., 2011, PLoS One 6, e24535).

### Example 3

### Administration of a compound of formula (I"), or a pharmaceutically acceptable salt, prevents tau phosphorylation.

Having shown that mechanical injury causes release of substance P after concussive injury, we then investigated whether a compound of formula (I) reduces tau hyperphosphorylation after concussive injury. Figure 3 shows the effects of a compound of formula (I") on tau phosphorylation after concussive injury. Note that concussive injury in the rat causes extensive tau phosphorylation (B) by 3 days after the concussive event compared to non-injured animals (A). The administration of a compound of formula (I") at 30 minutes after the induction of injury (1 mg/kg intravenously) results in almost complete inhibition of tau phosphorylation at the 3 day time point (C). Thus, administration of a compound of formula (I") prevents tau hyperphosphorylation and thus prevents the development of CTE.

### Example 4

### Concussion results in the greatest mechanical stress at the base of sulci

Having established that both release of substance P and accumulation of hyperphosphorylated tau occured following a concussive event, it remained to be shown why accumulation of hyperphosphorylated tau in human CTE was prominent at the base of the brain sulci. We have shown in example two that substance P release occurs in response to stimulation of mechanoreceptors on sensory nerve fibres. One highly innovative step in the current disclosure is the realization that in gyrencephalic animals, including man, sulci protect the brain cortex from mechanical injury by focusing the stress points to the base of the sulci. This is best illustrated in models of brain tissue that both incorporate and exclude sulci within them, and subsequently simulating the effects of mechanical stress (Cloots et al., 2008, Ann. Biomed. Eng. 36, 1203-1215). The simulation of the effects of mechanical strain typical of rotational acceleration in models replicating the brain tissue with and without sulci is shown in Figure 5. Higher mechanical stress is indicated as black. These results clearly show that the addition of sulci to the model focuses the stress to the base of the sulci, irrespective of the sulcus morphology. This mechanical stress pattern is remarkably similar to the post-mortem localisation of hyperphosphorylated tau reported in CTE and shown in Figure 1.

This example confirms that in a gyrencephalic brain, exposure to a mechanical concussive event will focus the mechanical forces to the base of the sulci, thereby preferentially activating mechanoreceptors on sensory nerves in that location. Example 2 has already shown that activation of mechanoreceptors on sensory nerve fibres will cause the perivascular release of substance P.

## Claims

1. Compound of Formula (I), or a pharmaceutically acceptable salt, or solvate thereof: 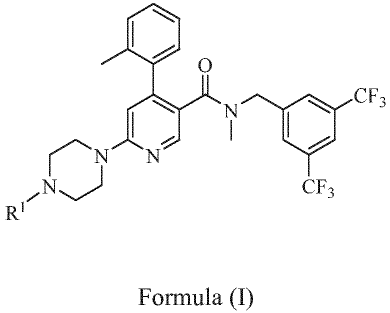 wherein R¹ is H or C₁₋₄ alkyl, or a pharmaceutically acceptable salt, or solvate thereof for use for preventing and/or treating chronic traumatic encephalopathy (CTE) in a subject.

2. The compound of Formula (I) for its use according to claim 1 wherein the compound of Formula (I) is represented by 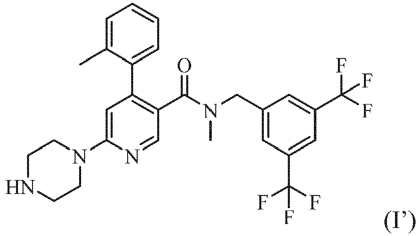 or a pharmaceutically acceptable salt, or solvate thereof.

3. The compound of Formula (I) for its use according to claim 1 wherein the compound of Formula (I) is represented by 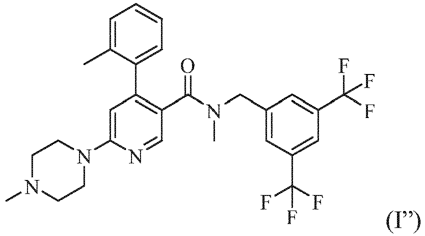 or a pharmaceutically acceptable salt, or solvate thereof.

4. The compound of Formula (I) for its use according to anyone of claims 1 to 3, wherein the prevention and/or treatment of CTE involves inhibiting the progression of tau hyperphosphorylation in said subject.

## Patentansprüche

1. Verbindung der Formel (I) oder ein pharmazeutisch unbedenkliches Salz oder Solvat davon: 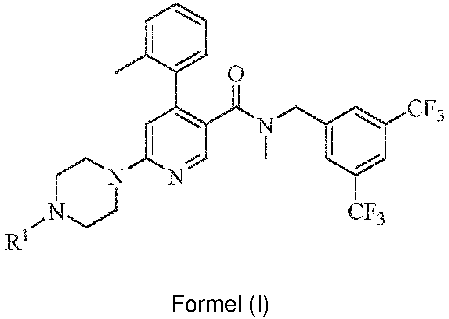
wobei R1 H oder C₁₋₄ -Alkyl oder ein pharmazeutisch unbedenkliches Salz oder Solvat davon ist,
zur Verwendung zur Verhütung und/oder Behandlung der chronischen traumatischen Enzephalopathie (CTE) bei einem Probanden.

2. Verbindung der Formel (I) zur Verwendung nach Anspruch 1, wobei die Verbindung der Formel (I) dargestellt ist durch 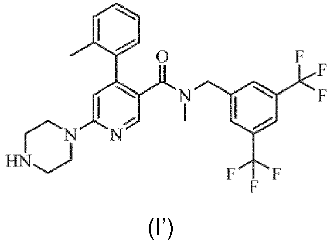 oder ein pharmazeutisch unbedenkliches Salz oder Solvat davon.

3. Verbindung der Formel (I) zur Verwendung nach Anspruch 1, wobei die Verbindung der Formel (I) dargestellt ist durch 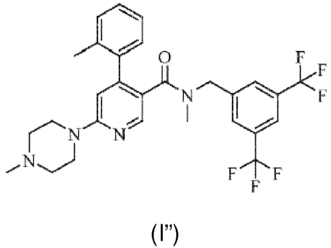 oder ein pharmazeutisch unbedenkliches Salz oder Solvat davon.

4. Verbindung der Formel (I) zur Verwendung nach einem der Ansprüche 1 - 3, wobei die Verhütung und/oder Behandlung der CTE die Hemmung der Progression einer Tau-Hyperphosphorylierung beim Probanden beinhaltet.

## Revendications

1. Composé de formule (I), ou un sel ou solvate pharmaceutiquement acceptable de celui-ci : 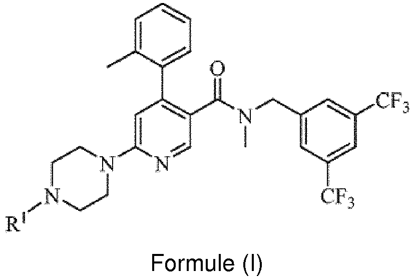
dans laquelle R¹ est H ou un alkyle en C₁ à C₄, ou un sel ou solvate pharmaceutiquement acceptable d'un tel composé,
pour une utilisation pour prévenir et/ou traiter une encéphalopathie traumatique chronique (CTE) chez un sujet.

2. Composé de formule (I) pour une utilisation selon la revendication 1, lequel composé de formule (I) est représenté par 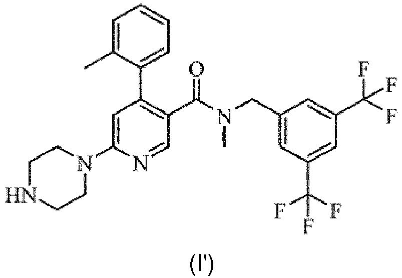 ou un sel ou solvate pharmaceutiquement acceptable de celui-ci.

3. Composé de formule (I) pour une utilisation selon la revendication 1, lequel composé de formule (I) est représenté par 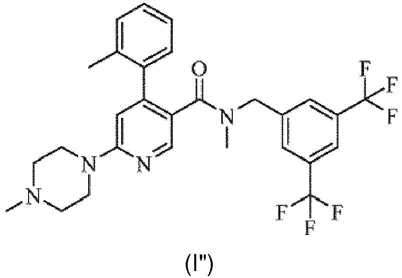 ou un sel ou solvate pharmaceutiquement acceptable de celui-ci.

4. Composé de formule (I) pour une utilisation selon l'une quelconque des revendications 1 à 3, où la prévention et/ou le traitement de la CTE comprend l'inhibition de la progression de l'hyperphosphorylation de tau chez ledit sujet.
